# EUROPEAN PATENT SPECIFICATION

(11) **EP 4 385 441 B1**
(45) Date of publication and mention of the grant of the patent: **13.08.2025**
(21) Application number: 23216572.0
(22) Date of filing: 14.12.2023
(51) Int. Cl.: A61B 18/14

(54) **CATHETER END EFFECTOR WITH WOVEN FLEX CIRCUIT**
KATHETERENDEFFEKTOR MIT GEWEBTER FLEXSCHALTUNG
EFFECTEUR D'EXTRÉMITÉ DE CATHÉTER AVEC CIRCUIT FLEXIBLE TISSÉ

(30) Priority: 15.12.2022 US 202263432838 P; 15.11.2023 US 202318510466
(43) Date of publication of application: 19.06.2024
(73) Proprietor: Biosense Webster (Israel) Ltd., 2066717 Yokneam (IL)
(72) Inventor: RORICK, Kevin, Irvine, 92618 (US)
(74) Representative: Carpmaels & Ransford LLP

(56) References cited:
- FR-A1- 3 117 763
- JP-A- 2019 063 546
- US-A1- 2015 351 652
- US-A1- 2021 153 932

## Description

### FIELD OF INVENTION

The present invention is directed to catheter, in particular, electrophysiology catheters for use in mapping and ablation of patient tissue.

### BACKGROUND

Cardiac arrhythmia, such as atrial fibrillation, occurs when regions of cardiac tissue abnormally conduct electric signals to adjacent tissue, thereby disrupting the normal cardiac cycle and causing asynchronous rhythm. Sources of undesired signals may be located in tissue of an atria or a ventricle. Unwanted signals may be conducted elsewhere through heart tissue where they can initiate or continue arrhythmia.

Procedures for treating arrhythmia include surgically disrupting the origin of the signals causing the arrhythmia, as well as disrupting the conducting pathway for such signals. By mapping the electrical properties of the endocardium and the heart volume, and selectively ablating cardiac tissue by application of energy, it may be possible to cease or modify the propagation of unwanted electrical signals from one portion of the heart to another. The ablation process may destroy the unwanted electrical pathways by formation of non-conducting regions of tissue.

In this two-step procedure, which includes mapping followed by ablation, electrical activity at points in the heart may be sensed and measured by advancing a catheter containing one or more electrical sensors into the heart and acquiring data at multiple points. These data may then be utilized to select the target areas at which ablation is to be performed.

For greater mapping resolution, it may be desirable for a mapping catheter to provide high-density signal maps through the use of several electrodes sensing electrical activity of tissue in an area on the order of a square centimeter.

Catheter end effectors that incorporate flexible circuits are known. However, electrical traces on the flexible circuits have been known to crack. The combination of flexible circuits and shape-memory underlying support structures, such as nitinol, is also known. The combination typically includes a nitinol support structure that is sandwiched between two flexible circuits that creates a three-layer structure which can minimize electrical traces cracking but not without added bulk and reduced flexibility which can hinder appropriate tissue contact for mapping and ablation.

Applicant recognized that there is a need for a mapping and ablation catheter end effector that utilizes the benefits of flex circuits while allowing flex circuits to remain flexible for appropriate tissue contact but with minimized risk of electrical traces cracking without significantly adding bulk.

FR3117763A1 relates to a medical device comprising a catheter that has a mobile distal portion comprising a body moving longitudinally inside a first lumen and a plurality of branches, a distal end of each branch comprising an electrically active zone and being able to move between a retracted position and a deployed position, in which said medical device can be electrically configured to deliver a first quantity of electrical energy to a first set of electrodes in the deployed position and a second quantity of electrical energy to a second set of electrodes in the retracted position.

Further prior art is disclosed in JP 2019 063546, US 2015/351652, US 2021/153932.

### SUMMARY OF THE DISCLOSURE

The present invention provides a catheter according to claim 1. Further embodiments of the invention are defined by the dependent claims.

A catheter providing an end effector with flex circuits woven onto structural members advantageously offers sufficient flexibility for improved tissue contact in mapping and ablation procedures but with appropriate structural support such that the risk of electrical traces cracking and breakage is reduced. With the ability to slide or adjust in slots formed in the structural members, the flex circuits have freedom to move and adjust in reducing the risk of electrical traces cracking while the structural members provide structural support and reinforcement so the end effector can hold shape without significant added bulk.

In some embodiments, a catheter for electrophysiology applications includes a shaft and an end effector. The shaft extends along a longitudinal axis to a distal end. The end effector is coupled to the distal end of the shaft and includes at least one loop member, a plurality of first electrodes and at least one flex circuit. The at least one loop member includes a pair of spines. The plurality of first electrodes is affixed to at least one spine of the at least one loop member. The at least one flex circuit is secured to the at least one spine of the at least one loop member, the at least one flex circuit including a plurality of second electrodes.

in some embodiments, the at least one loop member includes three loop members.

In some embodiments, the at least one loop member includes a pair of outer loop members and an inner loop member positioned radially inwardly of the pair of outer loop members relative to the longitudinal axis.

In some embodiments, the at least one loop member including a pair of inner loop members and an outer loop member positioned radially outwardly of the pair of inner loop members relative to the longitudinal axis.

In some embodiments, the catheter of any of claims 1 through 4, the at least one loop member including an elongate structural member.

In some embodiments, the elongate structural member includes a metallic material.

In some embodiments, the elongate structural member of the at least one at least one loop member includes a plurality of apertures.

In some embodiments, the at least one flex circuit being woven through the plurality of apertures.

In some embodiments, the plurality of first electrodes includes a plurality of front first electrodes disposed on a front of the at least one spine and a plurality of rear first electrodes disposed on a rear of the at least one spine.

In some embodiments, the plurality of front first electrodes being longitudinally offset from the plurality of rear first electrodes.

In some embodiments, the flex circuit including a flexible substrate, the plurality of second electrodes being affixed to the flexible substrate.

In some embodiments, the plurality of second electrodes includes a plurality of front second electrodes disposed on a front of the flexible substrate and a plurality of rear second electrodes disposed on a rear of the flexible substrate.

In some embodiments, the plurality of front second electrodes are longitudinally offset from the plurality of rear second electrodes.

In some embodiments, the plurality of first electrodes alternate longitudinally with the plurality of second electrodes on at least one side of the at least one spine.

In some embodiments, each of the plurality of first electrodes is directly laterally opposed by a corresponding one of the plurality of second electrodes.

In some embodiments, an end effector of a catheter comprises at least one loop member, a plurality of first electrodes and at least one flex circuit. The at least one loop member includes a pair of spines, the spines of each loop member extending parallel to a longitudinal axis. The plurality of first electrodes are affixed to at least one spine of the at least one loop member. The at least one flex circuit is secured to the at least one spine of the at least one loop member, the at least one flex circuit including a plurality of second electrodes.

In some embodiments, the plurality of first electrodes alternate longitudinally with the plurality of second electrodes on at least one side of the at least one spine.

In some embodiments, each of the plurality of first electrodes is directly laterally opposed by a corresponding one of the plurality of second electrodes.

In some embodiments, an end effector of a catheter comprises at least one loop member, a plurality of first electrodes and at least one flex circuit. The at least one loop member include a pair of spines, the spines of each loop member extending parallel to a longitudinal axis, and at least one spine of the at least one at least one loop member including a plurality of apertures. The plurality of first electrodes are affixed to the at least one spine of the at least one loop member. The at least one flex circuit is woven through the plurality of apertures, the at least one flex circuit including a plurality of second electrodes.

In some embodiments, the flex circuit includes a flexible substrate, the plurality of second electrodes including a plurality of front second electrodes disposed on a front of the flexible substrate and a plurality of rear second electrodes disposed on a rear of the flexible substrate.

In some embodiments, a catheter for electrophysiology applications includes a shaft and an end effector. The shaft extends along a longitudinal axis to a distal end. The end effector is coupled to the distal end of the shaft. The end effector includes a loop member including a spine, and a flex circuit secured to the spine. The flex circuit includes an elongated substrate, a plurality of first substrate electrodes and a plurality of second substrate electrodes. The elongated substrate is configured with a first substrate side and a second substrate side opposing the first substrate side. The plurality of first substrate electrodes are disposed on the first substrate side and the plurality of second substrate electrodes are disposed on the second substrate side, each first substrate electrode being laterally offset from each second substrate electrode.

According to the invention, the spine is configured with slots and the flex circuit is woven through the slots.

In some embodiments, the spine includes a first spine side and a second spine side opposing the first spine side, and the flex circuit is woven through the slots such that the first substrate side is exposed on the first spine side and the second substrate side is exposed on the second spine side.

In some embodiments, the spine includes a first spine side and a second spine side opposing the first spine side, and the flex circuit is woven through the slots such that the first substrate electrodes are exposed on the first spine side and the second substrate electrodes are exposed on the second spine side.

In some embodiments, the spine includes a plurality of first spine electrodes on the first spine side and a plurality of second spine electrodes on the second spine side.

In some embodiments, the flex circuit is woven through the slots such that the first substrate electrodes are exposed on the first spine side and the second substrate electrodes are exposed on the second spine side, and the first substrate electrodes alternate with the first spine electrodes on the first spine side and the second substrate electrodes alternate with the second spine electrodes on the second spine side.

In some embodiments, each electrode of the first and second substrate electrodes is directly opposite of a different electrode of the first and second spine electrodes.

In some embodiments, each first substrate electrode is directly opposite of a different second spine electrode, each second substrate electrode is directly opposite of a different first spine electrode.

In some embodiments, the flex circuit is loosely woven through the slots such that one or more air gaps are defined between the substrate and the spine.

In some embodiments, the flex circuit is loosely woven through the slots such that at least one of the plurality of first substrate electrodes is not parallel with the spine.

In some embodiments, the spine includes nitinol.

In some embodiments, wherein the loop member generally lies in a plane.

### BRIEF DESCRIPTION OF THE DRAWINGS

The drawings and detailed description that follow are intended to be merely illustrative and are not intended to limit the scope of the invention as contemplated by the inventors.

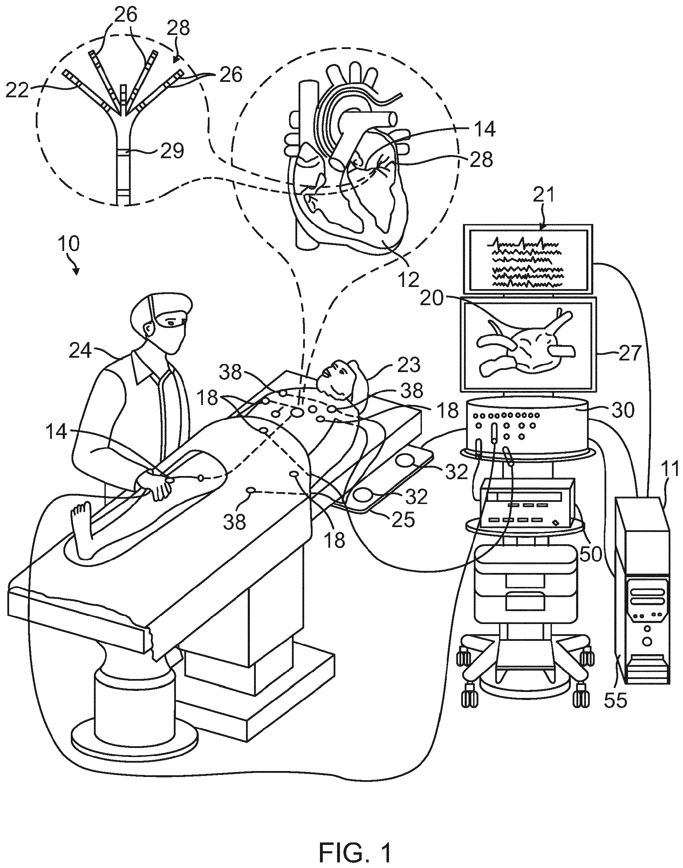

FIG. 1 depicts a schematic view of a medical procedure in which a catheter of a catheter assembly is inserted in a patient;

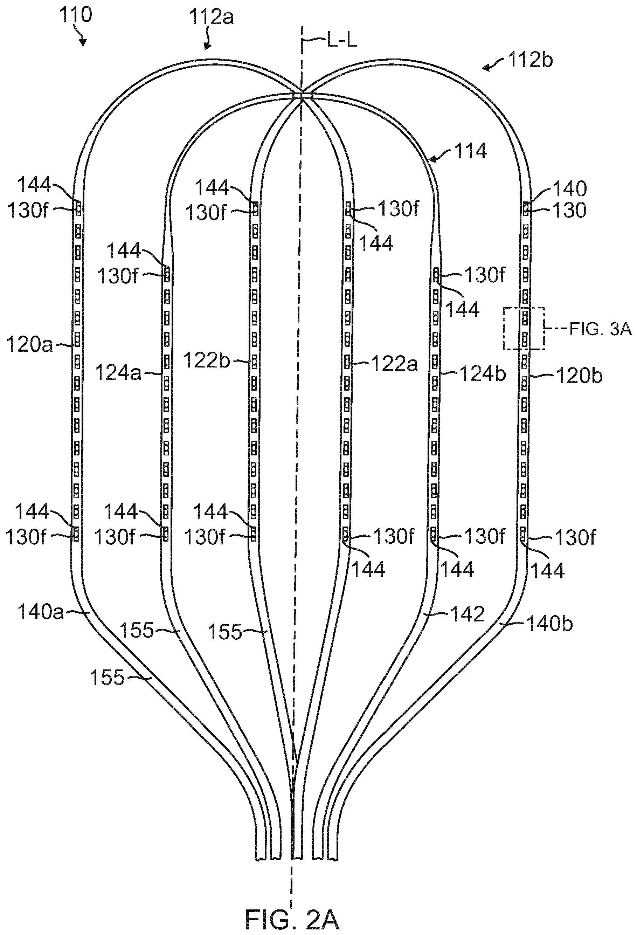

FIG. 2A depicts a front elevational view of an example of an end effector for use with the catheter of FIG. 1 and having a plurality of loop members configured according to one embodiment, each having a pair of spines, and further having a plurality of flex circuits woven through apertures of corresponding spines, with a plurality of electrodes provided by the flex circuits;

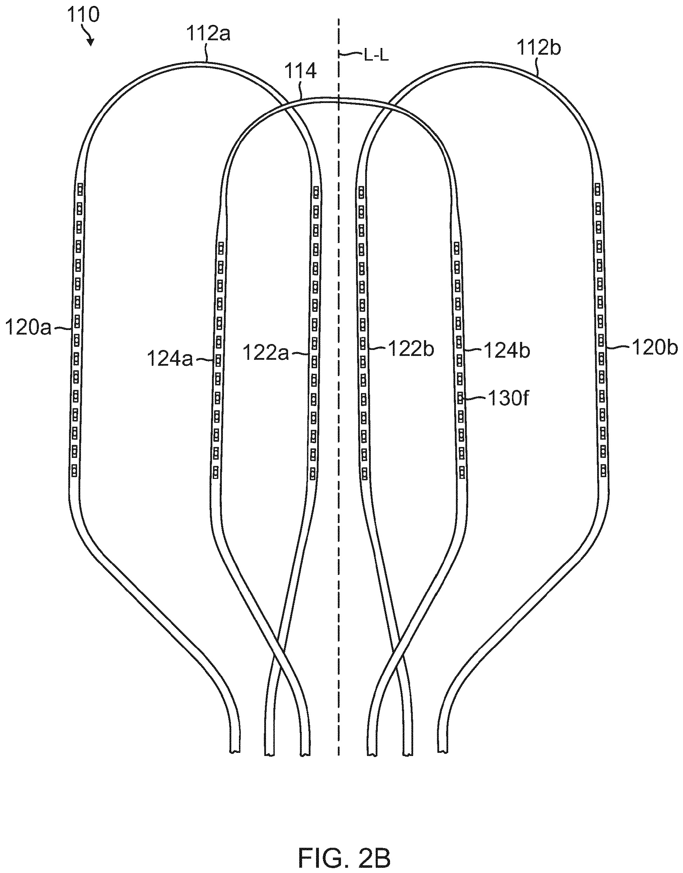

FIG. 2B depicts a front elevational view of another example of an end effector for use with the catheter of FIG. 1 and having a plurality of loop members configured according to another embodiment, each having a pair of spines, and further having a plurality of flex circuits woven through apertures of corresponding spines, with a plurality of electrodes provided by the flex circuits;

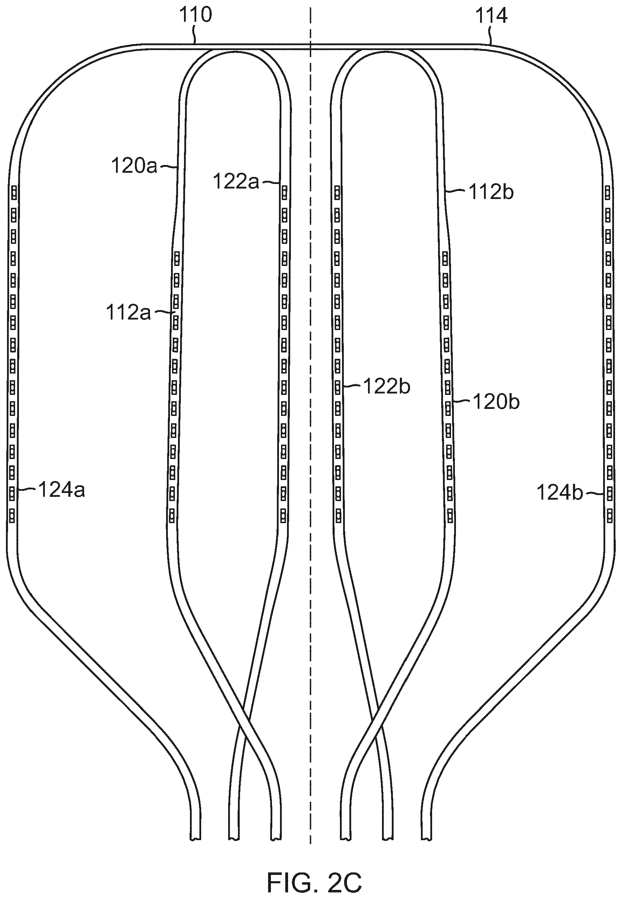

FIG. 2C depicts a front elevational view of another example of an end effector for use with the catheter of FIG. 1 and having a plurality of loop members configured according to yet another embodiment, each having a pair of spines, and further having a plurality of flex circuits woven through apertures of corresponding spines, with a plurality of electrodes provided by the flex circuits;

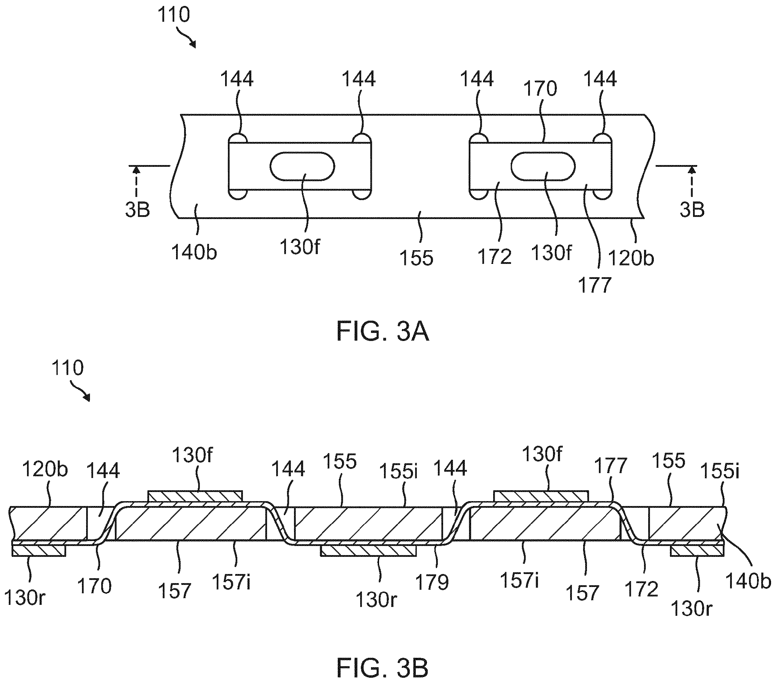

FIG. 3A depicts an enlarged front elevational view of a portion of the end effector of FIG. 2A, taken within area 3A in FIG. 2A;
FIG. 3B depicts a cross-sectional view of the end effector of FIG. 2A, taken along line 3B-3B in FIG. 3A;

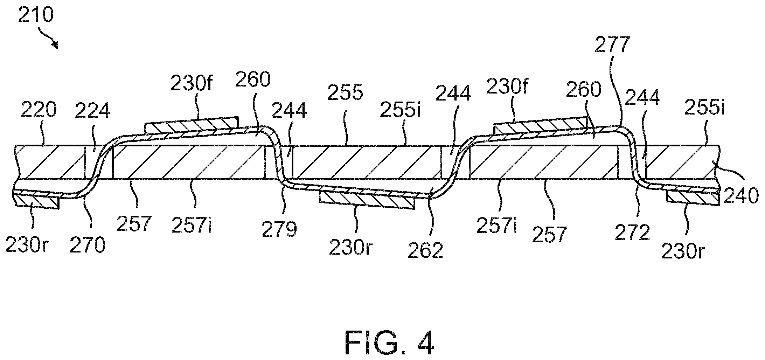

FIG. 4 depicts a cross-sectional view of another example of an end effector for use with the catheter of FIG. 1 and having at least one spine and at least one flex circuit woven through apertures of the at least one spine, with a plurality of electrodes provided by the flex circuit, the plurality of electrodes being non-parallel relative to the spine;

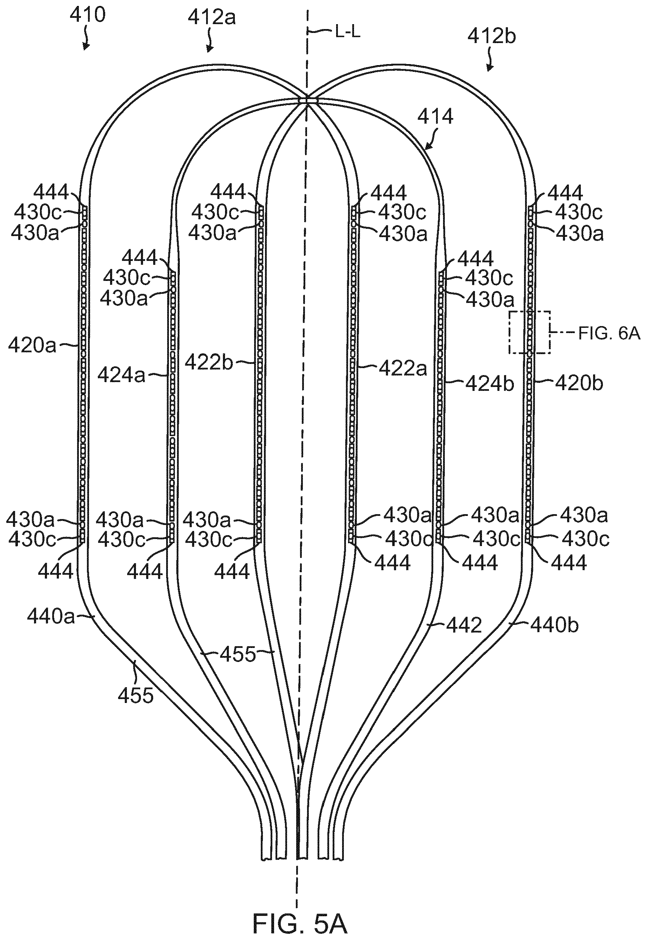

FIG. 5A depicts a front elevational view of an example of an end effector for use with the catheter of FIG. 1 and having a plurality of loop members configured according to one embodiment, each having a pair of spines, and further having a plurality of flex circuits woven through apertures of corresponding spines, with a first plurality of electrodes provided directly on the spines and a second plurality of electrodes provided by the flex circuits;

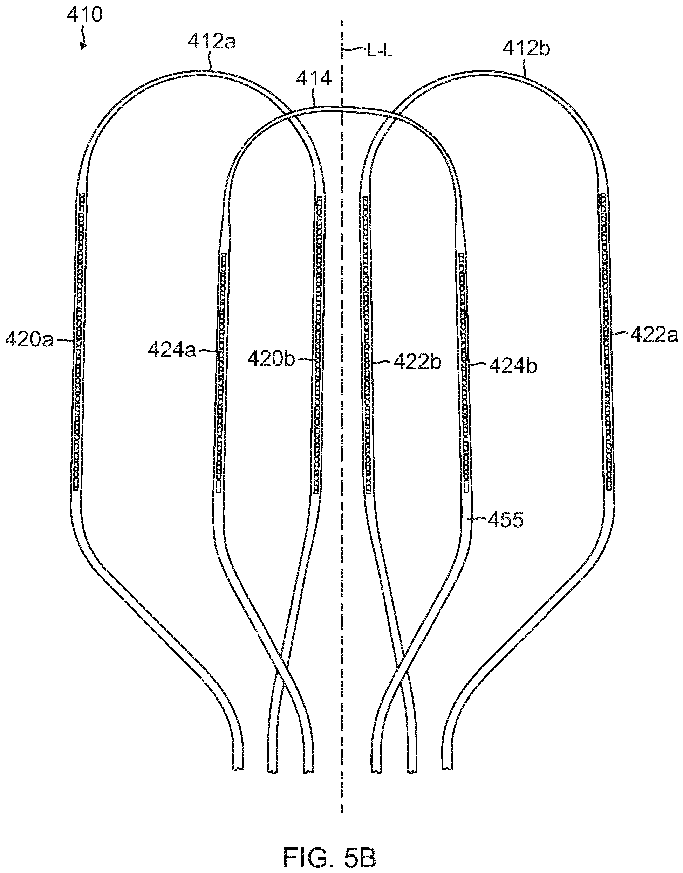

FIG. 5B depicts a front elevational view of an example of an end effector for use with the catheter of FIG. 1 and having a plurality of loop members configured according to another embodiment, each having a pair of spines, and further having a plurality of flex circuits woven through apertures of corresponding spines, with a first plurality of electrodes provided directly on the spines and a second plurality of electrodes provided by the flex circuits;

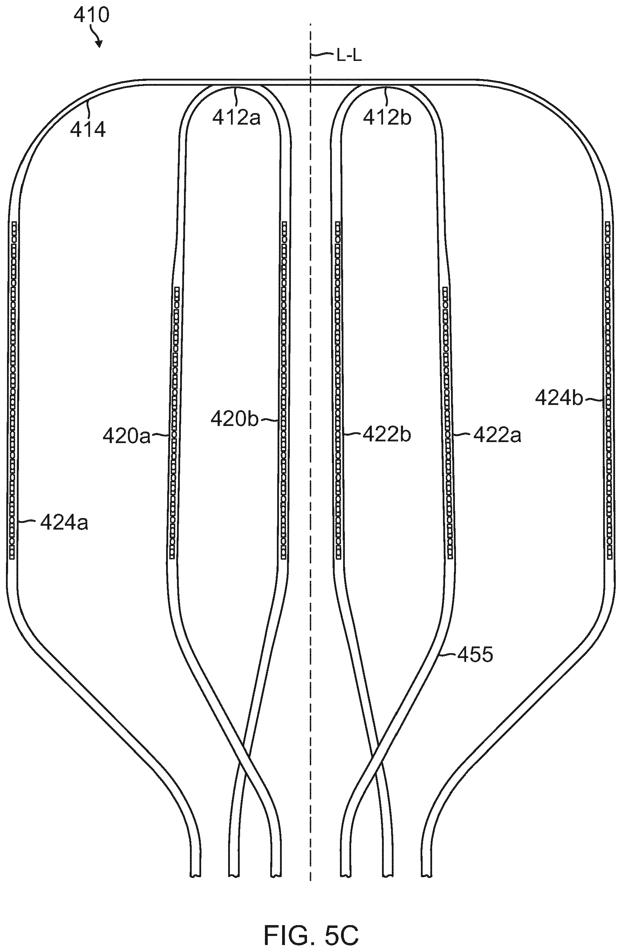

FIG. 5C depicts a front elevational view of an example of an end effector for use with the catheter of FIG. 1 and having a plurality of loop members configured according to yet another embodiment, each having a pair of spines, and further having a plurality of flex circuits woven through apertures of corresponding spines, with a first plurality of electrodes provided directly on the spines and a second plurality of electrodes provided by the flex circuits;

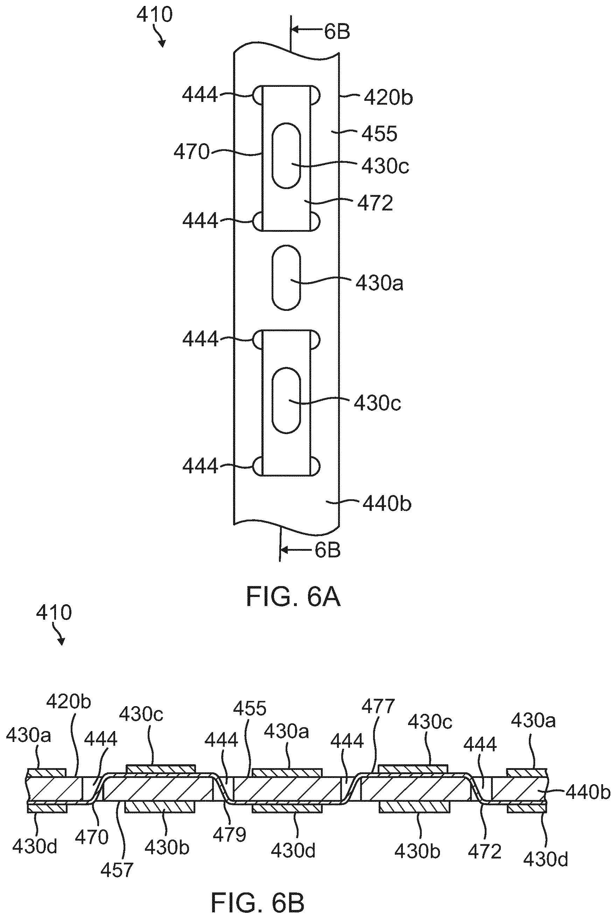

FIG. 6A depicts an enlarged front elevational view of a portion of the end effector of FIG. 5A, taken within area 6A in FIG. 5A;
FIG. 6B depicts a cross-sectional view of the end effector of FIG. 6A, taken along line 6B-6B in FIG. 6A; and

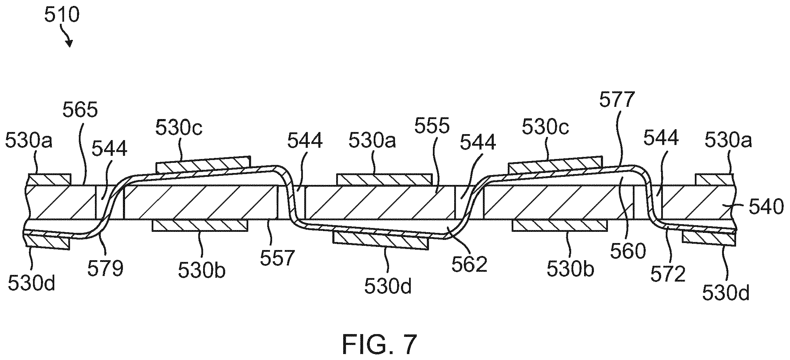

FIG. 7 depicts a cross-sectional view of another example of an end effector for use with the catheter of FIG. 1 and having at least one spine and at least one flex circuit woven through apertures of the at least one spine, with a first plurality of electrodes provided directly on the spines and a second plurality of electrodes provided by the flex circuits, the second plurality of electrodes being non-parallel relative to the spine.

### DETAILED DESCRIPTION FOR MODES OF CARRYING OUT THE INVENTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g. "about 90%" may refer to the range of values from 81% to 99%.

In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator whereas "distal" indicates a location further away to the operator or physician. Terms including "upper," "lower," "front," "rear," "above," "below," and the like are relative orientations, not absolute, and therefore are understood as not limiting or restrictive.

Any one or more of the teachings, expressions, versions, examples, etc. described herein may be combined with any one or more of the other teachings, expressions, versions, examples, etc. that are described herein. The following-described teachings, expressions, versions, examples, etc. should therefore not be viewed in isolation relative to each other. Various suitable ways in which the teachings herein may be combined will be readily apparent to those skilled in the pertinent art in view of the teachings herein. Such modifications and variations are intended to be included within the scope of the claims.

Example end effectors are illustrated and disclosed herein which are generally planar and include multiple electrodes that can be configured for mapping and/or ablation. The end effectors can be joined to a shaft with additional catheter components to form a mapping and/or ablation catheter through processes disclosed herein and processes similar to those known by a person skilled in the pertinent art. The example end effectors illustrated herein include variations and features that are combinable to form additional end effector designs as understood by a person skilled in the pertinent art.

### I. Overview of Example of a Catheter System

Reference is made to **FIG. 1** showing an example catheter-based electrophysiology mapping and ablation system (10). System (10) includes multiple catheters, which are percutaneously inserted by physician (24) through the patient's vascular system into a chamber or vascular structure of a heart (12). Typically, a delivery sheath catheter is inserted into the left or right atrium near a desired location in heart (12). Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter (14) that is configured for sensing IEGM is illustrated herein. Physician (24) brings a distal tip (28) of catheter (14) into contact with the heart wall for sensing a target site in heart (12). For ablation, physician (24) would similarly bring a distal end of an ablation catheter to a target site for ablating.

Catheter (14) is an exemplary catheter that includes one and preferably multiple electrodes (26) optionally distributed over a plurality of splines (22) at distal tip (28) and configured to sense the IEGM signals. Catheter (14) may additionally include a position sensor (29) embedded in or near distal tip (28) for tracking position and orientation of distal tip (28). Optionally and preferably, position sensor (29) is a magnetic based position sensor including three magnetic coils for sensing three-dimensional (3D) position and orientation.

Magnetic based position sensor (29) may be operated together with a location pad (25) including a plurality of magnetic coils (32) configured to generate magnetic fields in a predefined working volume. Real time position of distal tip (28) of catheter (14) may be tracked based on magnetic fields generated with location pad (25) and sensed by magnetic based position sensor (29). Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091.

System (10) includes one or more electrode patches (38) positioned for skin contact on patient (23) to establish location reference for location pad (25) as well as impedance-based tracking of electrodes (26). For impedance-based tracking, electrical current is directed toward electrodes (26) and sensed at electrode skin patches (38) so that the location of each electrode can be triangulated via the electrode patches (38). Details of the impedance-based location tracking technology are described in US Patent Nos. 7,536,218; 7,756,576; 7,848,787; 7,869,865; and 8,456,182.

A recorder (11) displays electrograms (21) captured with body surface ECG electrodes (18) and IEGMs captured with electrodes (26) of catheter (14). Recorder (11) may include pacing capability for pacing the heart rhythm and/or may be electrically connected to a standalone pacer.

System (10) may include an ablation energy generator (50) that is adapted to conduct ablative energy to one or more of electrodes at a distal tip of a catheter configured for ablating. Energy produced by ablation energy generator (50) may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) (30) is an interface configured to establish electrical communication between catheters, electrophysiological equipment, power supply and a workstation (55) for controlling operation of system (10). Electrophysiological equipment of system (10) may include for example, multiple catheters, location pad (25), body surface ECG electrodes (18), electrode patches (38), ablation energy generator (50), and recorder (11). Optionally and preferably, PIU (30) additionally includes processing capability for implementing real-time computations of location of the catheters and for performing ECG calculations.

Workstation (55) includes memory, processor unit with memory or storage with appropriate operating software loaded therein, and user interface capability. Workstation (55) may provide multiple functions, optionally including modeling the endocardial anatomy in three-dimensions (3D) and rendering the model or anatomical map (20) for display on a display device (27); displaying on display device (27) activation sequences (or other data) compiled from recorded electrograms (21) in representative visual indicia or imagery superimposed on the rendered anatomical map (20); displaying real-time location and orientation of multiple catheters within the heart chamber; and displaying on display device (2)7 sites of interest such as places where ablation energy has been applied. One commercial product embodying elements of the system (10) is available as the CARTO^{™} 3 System, available from Biosense Webster, Inc., 31A Technology Drive, Irvine, CA 92618.

### II. Examples of End Effectors with Woven Flex Circuits

In some procedures, it may be desirable to provide catheter (14) with a generally planar end effector having electrodes disposed on both sides of the end effector without necessarily having such electrodes be parallel to each other. It will be appreciated that such an asymmetric arrangement of electrodes may provide catheter (14) with improved mapping and/or ablating capabilities. Each of the examples of end effectors (110, 210) described below may function in such a manner.

### A. First Example of End Effector with Woven Flex Circuit

**FIG. 2A****,** **FIG. 3A** and **FIG. 3B** depict an example of an end effector (110) that may be readily incorporated into catheter (14) in place of distal tip (28). End effector (110) of the present example includes first and second outer loop members (112a, 112b) and an inner loop member (114), each extending distally from a base member or shaft (not shown), which itself extends along a longitudinal axis (L-L). Loop members (112a, 112b, 114) may also be referred to as paddles, loops, and/or electrode loop assemblies.

In the example shown, first and second outer loop members (112a, 112b) are disposed on either side of longitudinal axis (L-L), at least partially radially outwardly of third or central loop member (114). More particularly, outer loop members (112a, 112b) each include a corresponding outermost spine member (120a, 120b) and a corresponding innermost spine member (122a, 122b), while inner loop member (114) includes a pair of intermediate spine members (124a, 124b). Spine members (120a, 120b, 122a, 122b, 124a, 124b) may also be referred to as spines. In this regard, outermost spines (120a, 120b) are positioned radially outwardly of intermediate spines (124a, 124b) with respect to longitudinal axis (L-L); and innermost spines (122a, 122b) are positioned radially inwardly of intermediate spines (124a, 124b) with respect to longitudinal axis (L-L), at least within the frame of reference of **FIG. 2A****.** It will be appreciated that first outermost spine member (120a) is integral with first innermost spine member (122a) as part of first outer loop (112a) and that second outermost spine member (120b) is integral with second innermost spine member (122b) as part of second outer loop (112b). Similarly, first intermediate spine member (124a) is integral with second intermediate spine member (124b) as part of inner loop (114).

In some versions, spine members (120a, 120b, 122a, 122b, 124a, 124b) may be generally arranged along a transverse axis that is orthogonal to longitudinal axis (L-L) such that end effector (110) may generally reside within a plane defined by the transverse axis and longitudinal axis (L-L) and may therefore be generally planar. First outermost spine member (120a), first intermediate spine member (124a), and second innermost spine member (122b) are arranged on a first side of longitudinal axis (L-L); and second outermost spine member (120b), second intermediate spine member (124b), and first innermost spine member (122a) are arranged on a second side of longitudinal axis (L-L).

It will be appreciated that any other suitable configurations and/or arrangements of loop members (112a, 112b, 114) and/or spine members (120a, 120b, 122a, 122b, 124a, 124b) may be used. For example, in some versions, first outermost spine member (120a), first intermediate spine member (124a), and first innermost spine member (122a) may be arranged on the first side of longitudinal axis (L-L); and second outermost spine member (120b), second intermediate spine member (124b), and second innermost spine member (122b) may be arranged on a second side of longitudinal axis (L-L), as shown in FIG. 2B. In addition, or alternatively, first and second loop members (112a, 112b) may be disposed on either side of longitudinal axis (L-L), radially inwardly of third or central loop member (114), as shown in **FIG. 2C****.**

Loop members (112a, 112b, 114) also each include a corresponding plurality of sensing electrodes (130f, 130r) tissue regions that should be targeted for ablation. For example, electrodes (130f, 130r) may monitor electrical signals emanating from conductive endocardial tissues to pinpoint the location of aberrant conductive tissue sites that are responsible for an arrhythmia. By way of example only, electrodes (130f, 130r) may be configured and operable in accordance with at least some of the teachings of U.S. Pub. No. 2020/0345262, entitled "Mapping Grid with High Density Electrode Array," published November 5, 2020.

In the example shown, each loop member (112a, 112b, 114) includes a corresponding structural member (140a, 140b, 142). In this regard, each outer loop member (112a, 112b) has a corresponding elongated structural member (140a, 140b) extending continuously through the length of the respective outer loop member (112a, 112b) to and from the base member, while inner loop member (114) has an elongated structural member (142) extending continuously through the length of inner loop member (114) to and from the base member. Structural members (140a, 140b, 142) may also be referred to as loop structural members, spine frames, or frames. Structural members (140a, 140b, 142) can each include a biocompatible metal such as stainless steel, cobalt chromium, or nitinol, for example, with suitable elastic flexibility such as shape-memory. In the present example, each structural member (140a, 140b, 142) is in the form of a flat strip having a rectangular cross-sectional profile with a first or front outer surface (155) and an opposing second or rear outer surface (157).

Each structural member (140a, 140b, 142) of the present example includes a plurality of apertures in the form of slots (144) extending from the front outer surface (155) to the rear outer surface (157) of the corresponding structural member (140a, 140b, 142) and spaced apart (e.g., uniformly spaced apart) from each other along the corresponding spine members (120a, 120b, 122a, 122b, 124a, 124b).

In the example shown, each loop member (112a, 112b, 114) includes a corresponding pair of flexible printed circuit boards (also referred to as "PCBs" or "flex circuits") (170) secured to the corresponding spine members (120a, 120b, 122a, 122b, 124a, 124b), with each flex circuit (170) being routed along the corresponding spine member (120a, 120b, 122a, 122b, 124a, 124b) through each slot (144) of the corresponding structural member (140a, 140b, 142). In this regard, each flex circuit (170) is woven back-and-forth between the front outer side (155) of the corresponding structural member (140a, 140b, 142) and the rear outer side (157) of the corresponding structural member (140a, 140b, 142) in a serpentine manner. Each flex circuit (170) of the present example includes an elongate flexible substrate (172) and a plurality of front and rear electrodes (130f, 130r) secured to the respective flexible substrate (172) on respective front surface (177) and rear surface (179). By way of example only, each flex circuit (170) may be in the form of a substantially flat ribbon that is woven through the corresponding structural member (140a, 140b, 142). In some versions, each flex circuit (170) may be configured and operable in accordance with at least some of the teachings of U.S. Pub. No. 2017/0312022, entitled "Irrigated Balloon Catheter with Flexible Circuit Electrode Assembly," published November 2, 2017; and/or U.S. Pub. No. 2018/0071017, entitled "Ablation Catheter with a Flexible Printed Circuit Board," published March 15, 2018.

Front and rear electrodes (130f, 130r) of the present example are disposed on front and rear outer surfaces (177, 179), respectively, of the corresponding flexible substrate (172), and are spaced apart from each other along the corresponding flexible substrate (172) at uniform intervals. As best shown in FIG. 3B, front electrodes (130f) are longitudinally offset from rear electrodes (130r). More particularly, front electrodes (130f) disposed on front surfaces are arranged to alternate longitudinally with rear electrodes (130r) disposed on rear surfaces along the corresponding flexible substrates (172), such that at least one (e.g., each) front electrode (130f) disposed on a front surface (177) is longitudinally interposed between a corresponding pair of longitudinally-adjacent rear electrodes (130r) disposed on the corresponding rear surface (179) and is not directly laterally opposed by (e.g., laterally aligned with) any rear electrodes (130r) disposed on the corresponding rear surface (179); and such that at least one (e.g., each) rear electrode (130r) disposed on a rear surface (179) is longitudinally interposed between a corresponding pair of longitudinally-adjacent front electrodes (130f) disposed on the corresponding front surface (177) and is not directly laterally opposed by (e.g., laterally aligned with) any front electrodes (130f) disposed on the corresponding front surface (177).

In the example shown, each flex circuit (170) is woven back-and-forth between the front side (155) of the corresponding structural member (140a, 140b, 142) and the rear side (157) of the corresponding structural member (140a, 140b, 142) through the corresponding slots (144) such that each corresponding front electrode (130f) is on the front side (155) of the corresponding structural member (140a, 140b, 142) and such that each corresponding rear electrode (130r) is on the rear side (157) of the corresponding structural member (140a, 140b, 142).

In this manner, front electrodes (130f) may alternate with front electrodeless intervals 155i of the front side (155) longitudinally along the front side 155 of the corresponding structural member (140a, 140b, 142), and rear electrodes (130r) may alternate with rear electrodeless intervals 157i of the rear side 157 longitudinally along the rear side 157 of the corresponding structural member (140a, 140b, 142), such that at least one (e.g., each) front electrodeless interval (155i) on a front surface of a structural member (140a, 140b, 142) is longitudinally interposed between a corresponding pair of longitudinally-adjacent front electrodes (130f) disposed on the front surface (177) of the corresponding flexible substrate (172) and is directly laterally opposed by (e.g., laterally aligned with) a corresponding rear electrode (130r) disposed on the rear surface (179) of the corresponding flexible substrate (172); at least one (e.g., each) rear electrodeless interval (157i) on a rear surface (157) of a structural member (140a, 140b, 142) is longitudinally interposed between a corresponding pair of longitudinally-adjacent rear electrodes (130r) disposed on the rear surface (179) of the corresponding flexible substrate (172) and is directly laterally opposed by (e.g., laterally aligned with) a corresponding front electrode (130f) disposed on the front surface (177) of the corresponding flexible substrate (172); such that at least one (e.g., each) front electrode (130f) disposed on a front surface (177) of a flexible substrate (172) is longitudinally interposed between a corresponding pair of front electrodeless intervals (155i) on the front surface (155) of the corresponding structural member (140a, 140b, 142) and is directly laterally opposed by (e.g., laterally aligned with) a corresponding rear electrodeless interval (157i) on the rear surface (157) of the corresponding structural member (140a, 140b, 142); and such that at least one (e.g., each) rear electrode (130r) disposed on a rear surface (179) of a flexible substrate (172) is longitudinally interposed between a corresponding pair of rear electrodeless intervals (157i) disposed on the rear surface (157) of the corresponding structural member (140a, 140b, 142) and is directly laterally opposed by (e.g., laterally aligned with) a corresponding rear electrodeless interval (157i) on the rear surface (157) of the corresponding structural member (140a, 140b, 142). Thus, front and rear electrodes (130f, 130r) may be arranged asymmetrically relative to the plane defined by the transverse axis and longitudinal axis (L-L).

The end effector (110) comprising the flex circuits (170) that are woven onto the structural members (140a, 140, 142) with shape-memory advantageously offers sufficient flexibility for improved tissue contact in mapping and ablation procedures but with appropriate structural support such that the risk of electrical trace cracking and breakage is reduced. With the ability to slide or adjust in the slots 144, the flex circuits 170 are afforded a predetermined amount of freedom to move relative to the structural members (140a, 140b, 142) in reducing the risk of the electrical traces cracking while the structural members provide a predetermined amount of structural support and reinforcement so the end effector can hold shape without significant added bulk. Moreover, by carrying electrodes on both the front and rear surface of the flexible substrate (172), the woven flex circuits (170) conveniently provide the end effector (110) with electrodes on front and rear surfaces without requiring a flexible circuit to be affixed to each of the front and rear surfaces of the structural member.

With further reference to **FIG. 3A** and **FIG. 3B****,** the flex circuit (170) is woven in the corresponding structural member (140a, 140b, 142) in a manner such that the front side (177) of the substrate (172) is exposed on the front side (155) of the structural member and the rear side (179) of the substrate (172) is exposed on the rear side (157) of the structural member. Moreover, the front electrodes (130f) are outwardly-facing and exposed on the front side (155) of the structural member and the rear electrodes (130r) are outwardly-facing and exposed on the rear side (157) of the structural member.

It is also understood that the length of each flex circuit (170) woven on the corresponding structural members may be of uniform length or the length may vary between different flex circuits (170).

In the example shown in FIG. 3B, each flex circuit (170) is more tightly woven relative to the corresponding structural member (140a, 140b, 142) so as to make contact and press against the front and rear surfaces (155, 157) of the corresponding structural member (140a, 140b, 142), such that front electrodes (130f) are each oriented generally parallel to the corresponding laterally-opposed rear surface (157), and such that rear electrodes (130r) are each oriented generally parallel to the corresponding laterally-opposed front surface 155. In some other versions as discussed further below, each flex circuit (170) may be more loosely woven relative to the corresponding structural member (140a, 140b, 142) so as to bow outwardly away from the front and rear surfaces (155, 157) of the corresponding structural member (140a, 140b, 142), such that front electrodes (130f) may each be oriented generally obliquely relative to the corresponding laterally-opposed rear surfaces (157), and such that rear electrodes (130r) may each be oriented generally obliquely relative to the corresponding laterally-opposed front surfaces (155).

### B. Second Example of End Effector with Woven Flex Circuit

**FIG. 4** depicts another example of an end effector (210) that may be readily incorporated into catheter (14) in place of distal tip (28). End effector (210) of the present example is similar to end effector (110) described above, except as otherwise described below. In this regard, end effector (210) may include a plurality of loop members (not shown), each including a corresponding pair of spine members (220) (one shown), a corresponding plurality of sensing electrodes (230f, 230r), and a corresponding structural member (240), each including a plurality of apertures in the form of slots (244) extending from the front outer surface (255) to the rear outer surface (257) of the corresponding structural member (240) and spaced apart from each other along the corresponding spine members (220). In the present example, each structural member (240) is in the form of a flat strip having a rectangular cross-sectional profile.

In the example shown, each loop member further includes a corresponding pair of flex circuits (270) (one shown) secured to the corresponding spine members (220), with each flex circuit (270) being routed along the corresponding spine member (220) through each slot (244) of the corresponding structural member (240) in a manner similar to that described above. Each flex circuit (270) of the present example includes an elongate flexible substrate (272) and a plurality of front and rear electrodes (230f, 230r) secured to the respective flexible substrate (272). Each flex circuit (270) may be in a substantially flat ribbon form as described above with respect to flex circuits (170). In some versions, each flex circuit (270) may be configured and operable in accordance with at least some of the teachings of U.S. Pub. No. 2017/0312022, entitled "Irrigated Balloon Catheter with Flexible Circuit Electrode Assembly," published November 2, 2017; and/or U.S. Pub. No. 2018/0071017, entitled "Ablation Catheter with a Flexible Printed Circuit Board," published March 15, 2018.

In the example shown, each flex circuit (270) is loosely woven relative to the corresponding structural member (240) so as to bend (e.g., bow) outwardly away from the front and rear surfaces (255, 257) of the corresponding structural member (240) generally avoiding contact and forming space gaps (260, 262) between the substrate 272 and the structural member 240. As shown, such bending of each flex circuit (270) may cause the corresponding front and rear electrodes (230f, 230r) disposed on the corresponding flexible substrate (272) to be non-parallel relative to the front surface (255, 257) of the corresponding structural member (240). In this manner, front and rear electrodes (230f, 230r) may be asymmetric relative to the plane defined by the transverse axis and longitudinal axis (L-L), with the front and rear electrode (230f, 230r) being non-parallel to the plane.

### C. Third Example of End Effector with Woven Flex Circuit

**FIG. 5A****,** **FIG. 6A,** and **FIG. 6B** depict an example of an end effector (410) that may be readily incorporated into catheter (14) in place of distal tip (28). End effector (410) of the present example includes first and second outer loop members (412a, 412b) and an inner loop member (414), each extending distally from a base member or shaft (not shown), which itself extends along a longitudinal axis (L-L). Loop members (412a, 412b, 414) may also be referred to as paddles, loops, and/or electrode loop assemblies.

In the example shown, first and second outer loop members (412a, 412b) are disposed on either side of longitudinal axis (L-L), at least partially radially outwardly of third or central loop member (414). More particularly, outer loop members (412a, 412b) each include a corresponding outermost spine member (420a, 420b) and a corresponding innermost spine member (422a, 422b), while inner loop member (414) includes a pair of intermediate spine members (424a, 424b). Spine members (420a, 420b, 422a, 422b, 424a, 424b) may also be referred to as spines. In this regard, outermost spines (420a, 420b) are positioned radially outwardly of intermediate spines (424a, 424b) with respect to longitudinal axis (L-L); and innermost spines (422a, 422b) are positioned radially inwardly of intermediate spines (424a, 424b) with respect to longitudinal axis (L-L), at least within the frame of reference of **FIG. 5A** It will be appreciated that first outermost spine member (420a) is integral with first innermost spine member (422a) as part of first outer loop (412a) and that second outermost spine member (420b) is integral with second innermost spine member (422b) as part of second outer loop (412b). Similarly, first intermediate spine member (424a) is integral with second intermediate spine member (424b) as part of inner loop (114).

In some versions, spine members (420a, 420b, 422a, 422b, 424a, 424b) may be generally arranged along a transverse axis that is orthogonal to longitudinal axis (L-L) such that end effector (410) may generally reside within a plane defined by the transverse axis and longitudinal axis (L-L) and may therefore be generally planar. First outermost spine member (420a), first intermediate spine member (424a), and second innermost spine member (422b) are arranged on a first side of longitudinal axis (L-L); and second outermost spine member (420b), second intermediate spine member (424b), and first innermost spine member (422a) are arranged on a second side of longitudinal axis (L-L).

It will be appreciated that any other suitable configurations and/or arrangements of loop members (412a, 412b, 414) and/or spine members (420a, 420b, 422a, 422b, 424a, 424b) may be used. For example, in some versions, first outermost spine member (420a), first intermediate spine member (424a), and first innermost spine member (422a) may be arranged on the first side of longitudinal axis (L-L); and second outermost spine member (420b), second intermediate spine member (424b), and second innermost spine member (122b) may be arranged on a second side of longitudinal axis (L-L), as shown in **FIG. 5B****.** In addition, or alternatively, first and second loop members (412a, 412b) may be disposed on either side of longitudinal axis (L-L), radially inwardly of third or central loop member (414), as shown in **FIG. 5C****.**

Loop members (412a, 412b, 414) also each include a corresponding plurality of sensing electrodes (430a, 430b, 430c, 430d), including a corresponding plurality of first (or front spine) electrodes (430a) and second (or rear spine) electrodes (430b) secured to the respective spine members (420a, 420b, 422a, 422b, 424a, 424b), and a corresponding plurality of third (or front flex circuit) electrodes (430c) and fourth (or rear flex circuit) electrodes (430d) described in greater detail below. Electrodes (430a, 430b, 430c, 430d) may be configured to provide electrophysiology (EP) mapping, such as to identify tissue regions that should be targeted for ablation. For example, electrodes (430a, 430b, 430c, 430d) may monitor electrical signals emanating from conductive endocardial tissues to pinpoint the location of aberrant conductive tissue sites that are responsible for an arrhythmia. By way of example only, electrodes (430a, 430b, 430c, 430d) may be configured and operable in accordance with at least some of the teachings of U.S. Pub. No. 2020/0345262, entitled "Mapping Grid with High Density Electrode Array," published November 5, 2020.

In the example shown, each loop member (412a, 412b, 414) includes a corresponding structural member (440a, 440b, 442). In this regard, each outer loop member (412a, 412b) has a corresponding elongated structural member (440a, 440b) extending continuously through the length of the respective outer loop member (412a, 412b) to and from the base member, while inner loop member (414) has an elongated structural member (442) extending continuously through the length of inner loop member (414) to and from the base member. Structural members (440a, 440b, 442) may also be referred to as loop structural members, spine frames, or frames. Structural members (440a, 440b, 442) can each include a biocompatible metal such as stainless steel, cobalt chromium, or nitinol, for example, with suitable elastic flexibility such as shape-memory. In the present example, each structural member (440a, 440b, 442) is in the form of a flat strip having a rectangular cross-sectional profile.

First and second electrodes (430a, 430b) of the present example are disposed on front and rear outer surfaces (455, 457), respectively, of the corresponding structural member (440a, 440b, 442), and are spaced apart from each other along the corresponding spine members (420a, 420b, 422a, 422b, 424a, 424b) at uniform intervals. As best shown in **FIG. 6B****,** first electrodes (430a) are longitudinally offset from second electrodes (430b). More particularly, first electrodes (430a) disposed on front surfaces (455) are arranged to alternate longitudinally with second electrodes (430b) disposed on rear surfaces (457) along the corresponding spine members (420a, 420b, 422a, 422b, 424a, 424b), such that at least one (e.g., each) first electrode (430a) disposed on a front surface (455) is longitudinally interposed between a corresponding pair of longitudinally-adjacent second electrodes (430b) disposed on the corresponding rear surface (457) and is not directly laterally opposed by (e.g., laterally aligned with) any second electrodes (430b) disposed on the corresponding rear surface (457); and such that at least one (e.g., each) second electrode (430b) disposed on a rear surface (457) is longitudinally interposed between a corresponding pair of longitudinally-adjacent first electrodes (430a) disposed on the corresponding front surface (455) and is not directly laterally opposed by (e.g., laterally aligned with) any first electrodes (430a) disposed on the corresponding front surface (455). Thus, first and second electrodes (430a, 430b) may be arranged asymmetrically relative to the plane defined by the transverse axis and longitudinal axis (L-L).

In some versions, each loop member (412a, 412b, 414) may further include a corresponding coating or cover (not shown), with the corresponding structural member (440a, 440b, 442) underlying the corresponding coating/cover and with the corresponding plurality of first and second electrodes (430a, 430b) disposed on front and rear outer surfaces, respectively, of the corresponding coating/cover. Alternatively, first and second electrodes (430a, 430b) may be otherwise exposed relative to the coating/cover. In either of these arrangements, such coatings/covers may each be electrically insulative, and/or may each include a polymeric material such as polyurethane, for example.

Each structural member (440a, 440b, 442) of the present example includes a plurality of apertures in the form of slots (444) extending from the front outer surface (455) to the rear outer surface (457) of the corresponding structural member (440a, 440b, 442) and spaced apart from each other along the corresponding spine members (420a, 420b, 422a, 422b, 424a, 424b). In this regard, each slot (444) is longitudinally interposed between a corresponding pair of longitudinally-adjacent first and second electrodes (430a, 430b) such that each slot (444) is longitudinally flanked by a corresponding first electrode (430a) and by a corresponding second electrode (430b). In cases where each loop member (412a, 412b, 414) includes a corresponding cover, the corresponding cover may include a plurality of slots aligned with slots (444) such that the covers may not interfere with the functionality of slots (444) described below.

In the example shown, each loop member (412a, 412b, 414) further includes a corresponding pair of flexible printed circuit boards (also referred to as "PCBs" or "flex circuits") (170) secured to the corresponding spine members (420a, 420b, 422a, 422b, 424a, 424b), with each flex circuit (470) being routed along the corresponding spine member (420a, 420b, 422a, 422b, 424a, 424b) through each slot (444) of the corresponding structural member (440a, 440b, 442). In this regard, each flex circuit (470) is woven back-and-forth between a front side (455) of the corresponding structural member (440a, 440b, 442) and a rear side (457) of the corresponding structural member (440a, 440b, 442) in a serpentine manner. Each flex circuit (470) of the present example includes an elongate flexible substrate (472) and a plurality of third (or front) and fourth (or rear) electrodes (430c, 430d) secured to the respective flexible substrate (472). By way of example only, each flex circuit (470) may be in the form of a substantially flat ribbon that is woven through the corresponding structural member (440a, 440b, 442). In some versions, each flex circuit (470) may be configured and operable in accordance with at least some of the teachings of U.S. Pub. No. 2017/0312022, entitled "Irrigated Balloon Catheter with Flexible Circuit Electrode Assembly," published November 2, 2017; and/or U.S. Pub. No. 2018/0071017, entitled "Ablation Catheter with a Flexible Printed Circuit Board," published March 15, 2018.

Third and fourth electrodes (430c, 430d) of the present example are disposed on front and rear outer surfaces (477, 479), respectively, of the corresponding flexible substrate (472), and are spaced apart from each other along the corresponding flexible substrate (472) at uniform intervals. As best shown in **FIG. 6B****,** third electrodes (430c) are longitudinally offset from fourth electrodes (430d). More particularly, third electrodes (430c) disposed on front surfaces (477) are arranged to alternate longitudinally with fourth electrodes (430d) disposed on rear surfaces (479) along the corresponding flexible substrates (472), such that at least one (e.g., each) third electrode (430c) disposed on a front surface (477) is longitudinally interposed between a corresponding pair of longitudinally-adjacent fourth electrodes (430d) disposed on the corresponding rear surface (477) and is not directly laterally opposed by (e.g., laterally aligned with) any fourth electrodes (430d) disposed on the corresponding rear surface (479); and such that at least one (e.g., each) fourth electrode (430d) disposed on a rear surface (479) is longitudinally interposed between a corresponding pair of longitudinally-adjacent third electrodes (430c) disposed on the corresponding front surface (477) and is not directly laterally opposed by (e.g., laterally aligned with) any third electrodes (430c) disposed on the corresponding front surface (477).

In the example shown, each flex circuit (470) is woven back-and-forth between the front side (455) of the corresponding structural member (440a, 440b, 442) and the rear side (457) of the corresponding structural member (440a, 440b, 442) through the corresponding slots (444) such that each corresponding third electrode (430c) is on the front side (455) of the corresponding structural member (440a, 440b, 442) and such that each corresponding fourth electrode (430d) is on the rear side (457) of the corresponding structural member (440a, 440b, 442).

In this manner, first and third electrodes (430a, 430c) may alternate with each other longitudinally along the front side (455) of the corresponding structural member (440a, 440b, 442), and second and fourth electrodes (430b, 430d) may alternate with each other longitudinally along the rear side (457) of the corresponding structural member (440a, 440b, 442), such that at least one (e.g., each) first electrode (430a) disposed on a front surface (455) of a structural member (440a, 440b, 442) is longitudinally interposed between a corresponding pair of longitudinally-adjacent third electrodes (430c) disposed on the front surface (455) of the corresponding flexible substrate (472) and is directly laterally opposed by (e.g., laterally aligned with) a corresponding fourth electrode (430d) disposed on the rear surface (457) of the corresponding flexible substrate (472); at least one (e.g., each) second electrode (430b) disposed on a rear surface (457) of a structural member (440a, 440b, 442) is longitudinally interposed between a corresponding pair of longitudinally-adjacent fourth electrodes (430d) disposed on the rear surface (477) of the corresponding flexible substrate (472) and is directly laterally opposed by (e.g., laterally aligned with) a corresponding third electrode (430c) disposed on the front surface (477) of the corresponding flexible substrate (472); such that at least one (e.g., each) third electrode (430c) disposed on a front surface (477) of a flexible substrate (472) is longitudinally interposed between a corresponding pair of longitudinally-adjacent first electrodes (430a) disposed on the front surface (477) of the corresponding structural member (440a, 440b, 442) and is directly laterally opposed by (e.g., laterally aligned with) a corresponding second electrode (430b) disposed on the rear surface (457) of the corresponding structural member (440a, 440b, 442); and such that at least one (e.g., each) fourth electrode (430d) disposed on a rear surface (477) of a flexible substrate (472) is longitudinally interposed between a corresponding pair of longitudinally-adjacent second electrodes (430b) disposed on the rear surface (457) of the corresponding structural member (440a, 440b, 442) and is directly laterally opposed by (e.g., laterally aligned with) a corresponding first electrode (430a) disposed on the rear surface (457) of the corresponding structural member (440a, 440b, 442).

With further reference to FIG. 6A and FIG. 6B, in some embodiments, the flex circuits 470 are woven in the corresponding structural member (440a, 440b, 442) in a manner such that the front side (477) of the substrate (472) is exposed on the front side (455) of the structural member and the rear side (479) of the substrate (472) is exposed on the rear side (457) of the structural member. Moreover, the front or third electrodes (430c) are outwardly-facing and exposed on the front side (455) of the structural member; the rear or fourth electrodes (430d) are outwardly-facing and exposed on the rear side (457) of the structural member; the third electrodes (430c) alternate with the first electrodes (430a) of the front side (455) of the structural member; and the fourth electrodes (430d) alternate with the second electrodes (430b) on the rear side (457) of the structural member. Furthermore, each first electrode (430a) is generally directly opposite of a different or corresponding fourth electrode (430d), and each second electrode is generally directly opposite of a different or corresponding third electrode.

It is also understood that the length of each flex circuit (170) woven on the corresponding structural members may be of uniform length or the length may vary between different flex circuits (170).

The end effector (410) comprising the flex circuits (470) that are woven onto the structural members (440a, 440, 442) with shape-memory advantageously offers sufficient flexibility for improved tissue contact in mapping and ablation procedures but with appropriate structural support such that the risk of electrical trace cracking and breakage is reduced. With the ability to slide or adjust in the slots 444, the flex circuits 470 are afforded a predetermined amount of freedom to move relative to the structural members (440a, 440b, 442) in reducing the risk of the electrical traces cracking while the structural members provide a predetermined amount of structural support and reinforcement so the end effector can hold shape without significant added bulk. Moreover, by carrying electrodes on both the front and rear surfaces of the flexible substrate (472) and electrodes on both the front and rear surfaces of the structural members (440a, 440b, 442a, 442b, 442), the woven flex circuits (170) conveniently provide the end effector (410) with electrodes on front and rear surfaces without requiring a flexible circuit to be affixed to each of the front and rear surfaces of the structural member.

In the example shown in FIG. 6B, each flex circuit (470) is more tightly woven relative to the corresponding structural member (440a, 440b, 442) so as to make contact and press against the front and rear surfaces (455, 457) of the corresponding structural member (440a, 440b, 442), such that third electrodes (430c) are each oriented generally parallel to the corresponding laterally-opposed second electrodes (430b), and such that fourth electrodes (430d) are each oriented generally parallel to the corresponding laterally-opposed first electrodes (430a). In some other versions, each flex circuit (470) may be more loosely woven relative to the corresponding structural member (440a, 440b, 442) so as to bow outwardly away from the front and rear surfaces (455, 457) of the corresponding structural member (440a, 440b, 442), such that third electrodes (430c) may each be oriented generally obliquely relative to the corresponding laterally-opposed second electrodes (430b), and such that fourth electrodes (430d) may each be oriented generally obliquely relative to the corresponding laterally-opposed first electrodes (430a).

### D. Fifth Example of End Effector with Woven Flex Circuit

**FIG. 7** depicts another example of an end effector (510) that may be readily incorporated into catheter (14) in place of distal tip (28). End effector (510) of the present example is similar to end effector (110) described above, except as otherwise described below. In this regard, end effector (510) may include a plurality of loop members (not shown), each including a corresponding pair of spine members (520) (one shown), a corresponding plurality of sensing electrodes (530a, 530b, 530c, 530d), and a corresponding structural member (540), each including a plurality of apertures in the form of slots (544) extending from the front outer surface (555) to the rear outer surface (557) of the corresponding structural member (540) and spaced apart from each other along the corresponding spine members (520). In the present example, each structural member (540) is in the form of a flat strip having a rectangular cross-sectional profile. First and second electrodes (530a, 530b) are secured to the respective spine members (220) in a manner similar to that described above.

In the example shown, each loop member further includes a corresponding pair of flex circuits (570) (one shown) secured to the corresponding spine members (520), with each flex circuit (570) being routed along the corresponding spine member (520) through each slot (544) of the corresponding structural member (540) in a manner similar to that described above. Each flex circuit (570) of the present example includes an elongate flexible substrate (572) and a plurality of third (or front flex circuit) electrodes (530c) and fourth (or rear flex circuit) electrodes (530d) secured to the respective flexible substrate (572). Each flex circuit (570) may be in a substantially flat ribbon form as described above with respect to flex circuits (570). In some versions, each flex circuit (570) may be configured and operable in accordance with at least some of the teachings of U.S. Pub. No. 2017/0312022, entitled "Irrigated Balloon Catheter with Flexible Circuit Electrode Assembly," published November 2, 2017; and/or U.S. Pub. No. 2018/0071017, entitled "Ablation Catheter with a Flexible Printed Circuit Board," published March 15, 2018.

In the example shown, each flex circuit (570) is loosely woven relative to the corresponding structural member (540) so as to bend (e.g., bow) outwardly away from the front and rear surfaces (555, 557) of the corresponding structural member (240) generally avoiding contact and forming space gaps (560, 562) between the substrate 572 and the structural member 240. As shown, such bending of each flex circuit (270) may cause the corresponding third and fourth electrodes (530c, 530d) disposed on the corresponding flexible substrate (572) to be non-parallel relative to the corresponding first and second electrodes (530a, 530b) disposed on the corresponding structural member (540). For example, third electrodes (530c) may each be oriented generally obliquely relative to the corresponding laterally-opposed second electrodes (530b), and fourth electrodes (530d) may each be oriented generally obliquely relative to the corresponding laterally-opposed first electrodes (530a). In this manner, each laterally-opposed pair of electrodes (530a, 530b, 530c, 530d) may be asymmetric relative to the plane defined by the transverse axis and longitudinal axis (L-L), with the first or second electrode (530a, 530b) of each pair being parallel to the plane and with the third or fourth electrode (530c, 530d) of each pair being non-parallel to the plane.

## Claims

1. A catheter (14) for electrophysiology applications, comprising:
(a) a shaft extending along a longitudinal axis to a distal end; and
(b) an end effector (110, 410) coupled to the distal end of the shaft, the end effector including:
(i) at least one loop member (110, 112a, 112b, 410, 412a, 412b) including a spine (120a, 120b, 122a, 122b, 124a, 124b, 420a, 420b, 422a, 422b, 424a, 424b),
(ii) a flex circuit (170, 470) secured to the spine, the flex circuit comprising:
(1) an elongated substrate (172, 472) with a first substrate side and a second substrate side opposing the first substrate side, and
(2) a plurality of first substrate electrodes (130f, 430c) disposed on the first substrate side (177, 477);
wherein the spine is configured with slots (144, 444) and the flex circuit is woven through the slots.

2. The catheter of claim 1, wherein the flex circuit further comprises a plurality of second substrate electrodes (130r, 430b) disposed on the second substrate side (179, 479), each first substrate electrode being laterally offset from each second substrate electrode.

3. The catheter of claim 2, wherein the spine has a first spine side (155, 455) and a second spine side (157, 457) opposing the first spine side, and the flex circuit is woven through the slots such that the first substrate electrodes are exposed on the first spine side and the second substrate electrodes are exposed on the second spine side.

4. The catheter of claim 3, wherein the spine includes a plurality of first spine electrodes (430a) on the first spine side and a plurality of second spine electrodes (430d) on the second spine side.

5. The catheter of claim 4, wherein the flex circuit is woven through the slots such that the first substrate electrodes are exposed on the first spine side and the second substrate electrodes are exposed on the second spine side, and the first substrate electrodes alternate with the first spine electrodes on the first spine side and the second substrate electrodes alternate with the second spine electrodes on the second spine side.

6. The catheter of claim 5, wherein each electrode of the first and second substrate electrodes is directly opposite of a different electrode of the first and second spine electrodes.

7. The catheter of claim 5, wherein each first substrate electrode is directly opposite of a different second spine electrode, each second substrate electrode is directly opposite of a different first spine electrode.

8. The catheter of any preceding claim, wherein the flex circuit is loosely woven through the slots such that either:
one or more air gaps are defined between the substrate and the spine; or
at least one of the plurality of first substrate electrodes is not parallel with the spine.

9. The catheter of any preceding claim, wherein the spine includes nitinol.

10. The catheter of any preceding claim, wherein the at least one loop member generally lies in a plane.

11. The catheter of claim 1, wherein the spine is one of a pair of spines, and wherein the end effector further includes a plurality of electrodes (530a, 530b) disposed on at least one spine of the at least one loop member.

12. The catheter of any preceding claim, the at least one loop member including three loop members (412a, 412b, 414).

13. The catheter of claim 12, the at least one loop member including a pair of outer loop members and an inner loop member positioned radially inwardly of the pair of outer loop members relative to the longitudinal axis, or a pair of inner loop members and an outer loop member positioned radially outwardly of the pair of inner loop members relative to the longitudinal axis.

14. The catheter of any preceding claim, the at least one loop member including an elongate structural member, optionally the elongate structural member including a metallic material.

15. The catheter of any preceding claim, the plurality of electrodes including a plurality of front electrodes (530a) disposed on a front of the at least one spine and a plurality of rear electrodes (530b) disposed on a rear of the at least one spine, optionally the plurality of front electrodes being longitudinally offset from the plurality of rear electrodes.

## Patentansprüche

1. Katheter (14) für elektrophysiologische Anwendungen, umfassend:
(a) einen Schaft, der sich entlang einer Längsachse bis zu einem distalen Ende erstreckt; und
(b) einen Endeffektor (110, 410), der mit dem distalen Ende des Schafts verbunden ist, wobei der Endeffektor Folgendes einschließt:
(i) mindestens ein Schlaufenelement (110, 112a, 112b, 410, 412a, 412b), das einen Dorn (120a, 120b, 122a, 122b, 124a, 124b, 420a, 420b, 422a, 422b, 424a, 424b) einschließt,
(ii) eine flexible Schaltung (170, 470), die an dem Dorn befestigt ist, die flexible Schaltung umfassend:
(1) ein längliches Substrat (172, 472) mit einer ersten Substratseite und einer zweiten Substratseite, die der ersten Substratseite gegenüberliegt, und
(2) eine Vielzahl von ersten Substratelektroden (130f, 430c), die auf der ersten Substratseite (177, 477) angeordnet sind;
wobei der Dorn mit Schlitzen (144, 444) versehen ist und die flexible Schaltung durch die Schlitze gewebt ist.

2. Katheter nach Anspruch 1, wobei die flexible Schaltung ferner eine Vielzahl von zweiten Substratelektroden (130r, 430b) umfasst, die auf der zweiten Substratseite (179, 479) angeordnet sind, wobei jede erste Substratelektrode seitlich von jeder zweiten Substratelektrode versetzt ist.

3. Katheter nach Anspruch 2, wobei der Dorn eine erste Dornseite (155, 455) und eine zweite Dornseite (157, 457) aufweist, die der ersten Dornseite gegenüberliegt, und die flexible Schaltung durch die Schlitze gewebt ist, sodass die ersten Substratelektroden auf der ersten Dornseite und die zweiten Substratelektroden auf der zweiten Dornseite freigelegt sind.

4. Katheter nach Anspruch 3, wobei der Dorn eine Vielzahl von ersten Dornenelektroden (430a) auf der ersten Seite des Dorns und eine Vielzahl von zweiten Dornenelektroden (430d) auf der zweiten Seite des Dorns einschließt.

5. Katheter nach Anspruch 4, wobei die flexible Schaltung so durch die Schlitze gewebt ist, dass die ersten Substratelektroden auf der ersten Dornseite und die zweiten Substratelektroden auf der zweiten Dornseite freiliegen und die ersten Substratelektroden sich mit den ersten Dornenelektroden auf der ersten Dornseite und die zweiten Substratelektroden sich mit den zweiten Dornenelektroden auf der zweiten Dornseite abwechseln.

6. Katheter nach Anspruch 5, wobei jede Elektrode der ersten und zweiten Substratelektrode direkt gegenüber einer anderen Elektrode der ersten und zweiten Dornenelektrode liegt.

7. Katheter nach Anspruch 5, wobei jede erste Substratelektrode direkt gegenüber einer anderen zweiten Dornenelektrode liegt, jede zweite Substratelektrode direkt gegenüber einer anderen ersten Dornenelektrode liegt.

8. Katheter nach einem der vorstehenden Ansprüche, wobei die flexible Schaltung locker durch die Schlitze gewebt ist, sodass entweder:
ein oder mehrere Luftspalten zwischen dem Substrat und dem Dorn definiert sind; oder
mindestens eine der mehreren ersten Substratelektroden nicht parallel zum Dorn ist.

9. Katheter nach einem der vorstehenden Ansprüche, wobei der Dorn Nitinol einschließt.

10. Katheter nach einem der vorstehenden Ansprüche, wobei das mindestens eine Schlaufenelement im Allgemeinen in einer Ebene liegt.

11. Katheter nach Anspruch 1, wobei der Dorn einer eines Paares von Dornen ist und wobei der Endeffektor ferner eine Vielzahl von Elektroden (530a, 530b) einschließt, die auf mindestens einem Dorn des mindestens einen Schlaufenelements angeordnet sind.

12. Katheter nach einem der vorstehenden Ansprüche, wobei das mindestens eine Schlaufenelement drei Schlaufenelemente (412a, 412b, 414) einschließt.

13. Katheter nach Anspruch 12, wobei das mindestens eine Schlaufenelement ein Paar äußerer Schlaufenelemente und ein inneres Schlaufenelement einschließt, das relativ zur Längsachse radial innerhalb des Paars äußerer Schlaufenelemente angeordnet ist, oder ein Paar innerer Schlaufenelemente und ein äußeres Schlaufenelement, das relativ zur Längsachse radial außerhalb des Paars innerer Schlaufenelemente angeordnet ist.

14. Katheter nach einem der vorstehenden Ansprüche, wobei das mindestens eine Schlaufenelement ein längliches Strukturelement einschließt, wobei das längliche Strukturelement optional ein metallisches Material einschließt.

15. Katheter nach einem der vorstehenden Ansprüche, wobei die Vielzahl von Elektroden eine Vielzahl von vorderen Elektroden (530a) einschließt, die auf einer Vorderseite des mindestens einen Dorns angeordnet sind, und eine Vielzahl von hinteren Elektroden (530b), die auf einer Rückseite des mindestens einen Dorns angeordnet sind, wobei optional die Vielzahl der vorderen Elektroden in Längsrichtung von der Vielzahl der hinteren Elektroden versetzt ist.

## Revendications

1. Cathéter (14) destiné à des applications électrophysiologiques, comprenant :
(a) un arbre s'étendant le long d'un axe longitudinal jusqu'à une extrémité distale ; et
(b) un effecteur (110, 410) accouplé à l'extrémité distale de l'arbre, l'effecteur comportant :
(i) au moins un élément de boucle (110, 112a, 112b, 410, 412a, 412b) comportant une nervure (120a, 120b, 122a, 122b, 124a, 124b, 420a, 420b, 422a, 422b, 424a, 424b),
(ii) un circuit flexible (170, 470) fixé à la nervure, le circuit flexible comprenant :
(1) un substrat allongé (172, 472) avec un premier côté de substrat et un second côté de substrat opposé au premier côté de substrat, et
(2) une pluralité de premières électrodes de substrat (130f, 430c) disposées sur le premier côté de substrat (177, 477) ;
dans lequel la nervure est conçue avec des fentes (144, 444) et le circuit flexible est tissé à travers les fentes.

2. Cathéter selon la revendication 1, dans lequel le circuit flexible comprend en outre une pluralité de secondes électrodes de substrat (130r, 430b) disposées sur le second côté de substrat (179, 479), chaque première électrode de substrat étant décalée latéralement par rapport à chaque seconde électrode de substrat.

3. Cathéter selon la revendication 2, dans lequel la nervure a un premier côté de nervure (155, 455) et un second côté de nervure (157, 457) opposé au premier côté de nervure, et le circuit flexible est tissé à travers les fentes de telle sorte que les premières électrodes de substrat sont exposées sur le premier côté de nervure et les secondes électrodes de substrat sont exposées sur le second côté de nervure.

4. Cathéter selon la revendication 3, dans lequel la nervure comporte une pluralité de premières électrodes de nervure (430a) sur le premier côté de nervure et une pluralité de secondes électrodes de nervure (430d) sur le second côté de nervure.

5. Cathéter selon la revendication 4, dans lequel le circuit flexible est tissé à travers les fentes de telle sorte que les premières électrodes de substrat sont exposées sur le première côté de nervure et les secondes électrodes de substrat sont exposées sur le second côté de nervure, et les premières électrodes de substrat alternent avec les premières électrodes de nervure sur le premier côté de nervure et les secondes électrodes de substrat alternent avec les secondes électrodes de nervure sur le seconde côté de nervure.

6. Cathéter selon la revendication 5, dans lequel chaque électrode des premières et secondes électrodes de substrat est directement opposée à une électrode différente des premières et secondes électrodes de nervure.

7. Cathéter selon la revendication 5, dans lequel chaque première électrode de substrat est directement opposée à une seconde électrode de nervure différente, chaque seconde électrode de substrat est directement opposée à une première électrode de nervure différente.

8. Cathéter selon l'une quelconque revendication précédente, dans lequel le circuit flexible est tissé de manière lâche à travers les fentes de telle sorte que soit :
un ou plusieurs espaces d'air sont définis entre le substrat et la nervure ; ou
au moins l'une parmi la pluralité de premières électrodes de substrat n'est pas parallèle à la nervure.

9. Cathéter selon l'une quelconque revendication précédente, dans lequel la nervure comporte du nitinol.

10. Cathéter selon l'une quelconque revendication précédente, dans lequel l'au moins un élément de boucle se trouve généralement dans un plan.

11. Cathéter selon la revendication 1, dans lequel la nervure est l'une d'une paire de nervures, et dans lequel l'effecteur comporte en outre une pluralité d'électrodes (530a, 530b) disposées sur au moins une nervure de l'au moins un élément de boucle.

12. Cathéter selon l'une quelconque revendication précédente, l'au moins un élément de boucle comportant trois éléments de boucle (412a, 412b, 414).

13. Cathéter selon la revendication 12, l'au moins un élément de boucle comportant une paire d'éléments de boucle externes et un élément de boucle interne positionné de manière radiale vers l'intérieur de la paire d'éléments de boucle externes par rapport à l'axe longitudinal, ou une paire d'éléments de boucle internes et un élément de boucle externe positionné de manière radiale vers l'extérieur de la paire d'éléments de boucle internes par rapport à l'axe longitudinal.

14. Cathéter selon l'une quelconque revendication précédente, l'au moins un élément de boucle comportant un élément structurel allongé, éventuellement l'élément structurel allongé comportant un matériau métallique.

15. Cathéter selon l'une quelconque revendication précédente, la pluralité d'électrodes comportant une pluralité d'électrodes avant (530a) disposées à l'avant de l'au moins une nervure et une pluralité d'électrodes arrière (530b) disposées à l'arrière de l'au moins une nervure, éventuellement la pluralité d'électrodes avant étant décalée de manière longitudinale par rapport à la pluralité d'électrodes arrière.
